Europäisches Patentamt

European Patent Office

Office européen des brevets

(11) Publication number : **0 324 197 B1**

# EUROPEAN PATENT SPECIFICATION

(45) Date of publication of patent specification :
**11.03.92 Bulletin 92/11**

(51) Int. Cl.⁵ : **A61K 35/78**

(21) Application number : **88202552.1**

(22) Date of filing : **15.11.88**

(54) **Process for extracting a dry residue from the gingko biloba leaves.**

The file contains technical information
submitted after the application was filed and
not included in this specification

(30) Priority : **18.12.87 CH 4943/87**

(43) Date of publication of application :
**19.07.89 Bulletin 89/29**

(45) Publication of the grant of the patent :
**11.03.92 Bulletin 92/11**

(84) Designated Contracting States :
**AT BE CH DE ES FR GB GR IT LI LU NL SE**

(56) References cited :
EP-A- 0 086 315
EP-A- 0 237 066
FR-A- 2 007 352

(56) References cited :
PHYTOCHEMISTRY, vol. 19, 1980, pages
1999-2002, Pergamon Press Ltd, GB; M. JOLY
et al.: "La 5'-méthoxybilobétine, une biflavone
extraite du Ginkgo biloba"
PHYTOCHEMISTRY, vol. 26, no. 10, 1987,
pages 2869-2870, Pergamon Journals Ltd, GB;
C. NASR et al.: "Quercetin coumaroyl
glucorhamnoside from Ginkgo biloba"
JOURNAL OF THE CHEMICAL SOCIETY, 1963,
pages 1477-1490; W. BAKER et al.: "The struc-
tures of the naturally occurring biflavonyls"

(73) Proprietor : **NUOVA LINNEA S.A.**
**CH-6595 Lavertezzo (TI) (CH)**

(72) Inventor : **Paracchini, Silvano**
**Via S. Maria**
**CH-6596 Gordola (CH)**

(74) Representative : **Marchi, Massimo et al**
**c/o Marchi & Mittler s.r.l. Viale Lombardia 20**
**I-20131 Milano (IT)**

EP 0 324 197 B1

## Description

The present invention relates to a process for isolating a dry extract from leaves of gingko biloba L. or salisburia adiantifolia Smith.

It is known that the above leaves contain compounds endowed with cerebral and peripheral vasomotor activity.

The German patent No. 1,767,098 teaches an extraction of the above compounds by a process which consists essentially in treating the leaves with an aqueous mixture of a lower alcohol or ketone. The thus obtained solution contains both the desired pharmacolgically-active compounds and some undesired compounds. Thus, the subsequent phase consists of eliminating the latter by extracting them with a lower halogen-substitited hydrocarbon.

It is thus clear that this process leads inevitably to the formation of a mixture of solvents which are difficult to recover and to re-cycle and which in any case require expensive operations with a severe environmental impact.

Moreover, the thus obtained product still contains some unidentified undesired compounds which prevent the preparation of stable injectable solutions.

The DE-A-2,117,429 intends to overcome the latter disadvantage by treating an aqueous solution of the product obtained according to the German patent No. 1,767,098 with ammonium sulphate and subsequently extracting the aqueous phase with a ketone. The ketone solution is then concentrated and the residue is treated with a lower alcohol.
The thus obtained solution is treated with a lead derivative and, after removing the precipitate by filtration, the solution is treated with ammonium sulphate and extracted with butanone. The organic phase is again treated with ammonium sulphate and dried.

It is clear that this second process, in addition to having all the drawbacks of the first, also has the drawback that it implies the use of toxic compounds, such as lead derivatives, whose use should be absolutely avoided, particularly in the preparation of drugs to be administered by injection.

Lastly, the complexity and the numerous manipulations required by the above processes are detrimental to the reproducibility of the yields and of the quality of the final product.

The need is therefore still strongly felt for a simple and economical process which avoids the use of toxic solvents or reactants and which is easily reproducible both as to the yield and quality of the final product.

It has now been found that celite, i.e. diatomite, may replace both the chlorine-substituted solvents and the lead derivatives. This was entirely unexpected and it has thus been possible to accomplish a simple and economical process which is easily reproducible both as to the yield and the quality of the final product.

The object of the present invention is, therefore, a process for isolating, a dry extract endowed with cerebral and peripheral vasomotor activity, from the leaves of gingko biloba L. which comprises treating the said leaves with an aqueous solution of a lower alcohol or ketone, concentrating the alcohol- or ketone-water solution, filtering the thus obtained concentrated aqueous suspension, treating this solution with an electrolyte and extracting with butanone, concentrating the organic phase to a dense residue, treating this residue with water, and drying the thus obtained aqueous suspension to a constant weight, characterized in that the concentration of the said alcohol- or ketone-water solution is performed in the presence of celite.

The gingko biloba leaves used in the process of this invention, preferably contain at least 0.45% by weight of flavonoids (as quercitin) tested according to DAB 9, pages 575 - 577, provided, however, that hyperoside has been substituted with quercitin as reference standard.

The first part of the process is carried out according to known techniques extracting the leaves with an aqueous solution of a lower alcohol or ketone. Examples of suitable alcohols and ketones are methyl, ethyl, propyl and butyl alcohol, acetone, butanone and methyl-propyl-ketone.

The extraction mixture, consisting of the suspension of the leaves in the extracting solvent, is filtered and pressed to complete dripping. Before being filtered it is preferably equilibrated at 25°C.

The clear solution thus obtained is concentrated in the presence of celite until a concentrated aqueous solution is obtained whose volume, preferably, is 1.5 - 1.6 times the weight of the treated leaves. The concentrated aqueous solution is then carefully filtered repeatedly on a celite panel.

An electrolyte is added to the thus obtained aqueous solution. Preferably the electrolyte is a chloride, a sulphate, a nitrate, or a bromide of an alkali or alkaline-earth metal or of ammonium. Even more preferably, the electrolyte is sodium chloride or ammonium sulphate.

The quantity of electrolyte to be added ranges preferably from 5 to 20% (w/v); even more preferably it is equal to 12 -13% (w/v).

The subsequent extraction with butanone is preferably carried out continuously at 25°C. The organic phase obtained after partition is anhydrified, filtered and concentrated to a dense residue under a reduced pressure.

The removal of the solvent is preferably completed by adding a volume of distilled water equal to the weight of the dense residue and drying the thus obtained suspension in vacuum at 45°C. The thus obtained product has the following properties:

    a) appearance: hygroscopic and deliquescent yellow-brown powder;

b) 4% solubility (w/v) in ethyl alcohol: as per standard

c) residual humidity according to K. Fischer: max 5%

d) ash residue: max 1%

e) identification:

e.1) the UV spectrum in methanol has 2 absorption peaks at 264 ± 2 nm and at 348 ± 2 nm, respectively;

e.2) the thin-layer chromatography performed on HPLTC MERCK plates, using a mixture of toluene/ethyl acetate/formic acid, shows a series of characteristic spots which are revealed by spraying with the diphenylboricester reagent and viewing the plate under a Wood light.

f) heavy metals: max 40 ppm

g) flavonoid units: from 7 to 8 (as quercitin)

h) organic solvents: max 0.01%

The following examples are given to illustrate the present invention without, however, limiting it in any way.

## Example 1

10 kg of gingko leaves containing 0.45% in flavonoids (as quercitin) have been loaded into a suitable extractor. The leaves have then been treated with 60 l of 65% (v/v) acetone under stirring at 60°C for 4.5 hours. The suspension has been cooled to 25°C and filtered on a double filter, squeezing the panel to complete dripping and washing the solid with 10 l of fresh aqueous acetone.

The filtrate has been separated, 200 g of celite have been added and the suspension has been concentrated at 45°C under a slightly reduced pressure to a final volume of 15 l. The suspension has been cooled to 25 - 28°C and filtered with great care on a celite panel washing with 1 l of $H_2O$ and pulping the solid again with care. The resulting aqueous solution has been treated at 25°C with 4 l of butanone and 2 kg of ammonium sulphate, the phases have been separated and the solution has been exhausted 3 times with 2 l of butanone each.

The organic layers have been combined, dried on anydrous sodium sulphate, filtered and concentrated at 60°, in vacuum, to an almost dry residue. The complete removal of the solvent has been obtained by adding water before completing the drying step.

Yield: 180 g of a dry extract having the following characteristics:

a) yellow brown powder deliquescent in air;

b) 4% solubility (w/v) in ethyl alcohol: as per standard

c) ashes: 0.25%

d) identification: as per standard

e) heavy metals: 20 ppm

f) titer: 7.6

g) organic solvents: not detectable.

## Example 2

10 kg of gingko leaves containing 0.49% in flavonoids (as quercitin) have been loaded into a suitable extractor. The leaves have then been treated with 60 l of 60% (v/v) ethanol under stirring at 60°C for 4.5 hours. The suspension has been cooled to 25°C and filtered on a double filter, squeezing the panel to complete dripping and processing the solid with 10 l of fresh aqueous ethanol.

The filtrate has been separated, 200 g of celite have been added and the suspension has been concentrated at 45°C under a slightly reduced pressure to a final volume of 15 l. The suspension has been cooled to 25 - 28°C and filtered with great care on a celite panel washing with 1 l of $H_2O$ and pulping the solid again with care. The resulting aqueous solution has been treated at 25°C with 4 l of butanone and 2 kg of ammonium sulphate, the phases have been separated and the solution has been exhausted 3 times with 2 l of butanone each.

The organic layers have been combined, dried on anydrous sodium sulphate, filtered and concentrated at 60°C, in vacuum, to an almost dry residue. The complete removal of the solvent has been obtained by adding water before completing the drying step.

Yield: 183 g of a dry extract having the following characteristics:

a) yellow brown powder deliquescent in air;

b) 4% solubility (w/v) in ethyl alcohol: as per standard

c) ashes: 0.3%

d) identitification: as per standard

e) heavy metals: 20 ppm

f) titer: 7.8

g) organic solvents: 0.005%

## Claims

1. A process for isolating a dry extract, endowed with cerebral and peripheral vasomotor activity, from the leaves of gingko biloba L. which comprises treating the said leaves with an aqueous solution of a lower alcohol or ketone, concentrating the alcohol- or ketone-water solution, filtering the thus obtained concentrated aqueous suspension, treating this solution with an electrolyte and extracting with butanone, concentrating the organic phase to a dense residue, treating this residue with water, and drying the thus obtained aqueous suspension to a constant weight, characterized in that the concentration of the said alcohol- or ketone-water solution is performed in the presence of celite.

2. A process according to claim 1, characterized in that the volume of the aqueous solution obtained by concentration of the alcohol- or ketone-water solution in the presence of celite is 1.5-1.6 times the weight of the extracted leaves.

## Patentansprüche

1. Verfahren zur Isolierung eines Trockenextraktes mit zerebraler und peripherer vasomotorischer Aktivität, aus den Gingko Biloba L. Blättern, wobein man die Blätter mit einer wässrigen Lösung eines niedrigen Alkohols oder Ketons behandelt, die Alkohol- oder Ketonlösung konzentriert, die so erhaltene konzentrierte wässrige Suspension filtriert, diese Lösung mit einem Elektrolyten behandelt und mit Butanon extrahiert, die organische Phase zu einem dicken Rückstand konzentriert, diesen Rückstand mit Wasser behandelt, und die so erhaltene wässrige Suspension zur Gewichtskonstanz trocknet, **dadurch gekennzeichnet**, daß die Alkohol- oder Keton-Waser-Lösung in Anwesenheit von Kieselgur konzentriert.

2. Verfahren nach Anspruch 1, **dadurch gekennzeichnet**, daß das Volumen der wässrigen Lösung, die durch Konzentrieren der Alkohol- oder Keton-Wasser-Lösung in Anweseheit von Kieselgur erhalten wurde, das 1,5 - 1,6 -fache des Gewichtes der extrahierten Blätter ist.

## Revendications

1. Procédé d'isolement d'un extrait sec, doué d'activité vasomotrice cérébrale et périphérique, des feuilles de gingko biloba L., comprenant le traitement desdites feuilles par une solution aqueuse d'un alcool inférieur ou d'une cétone, la concentration de la solution aqueuse alcoolique ou cétonique, la filtration de la suspension aqueuse concentrée ainsi obtenue, le traitement de cette solution par un électrolyte et l'extraction par la butanone, la concentration de la phase organique en un résidu dense, le traitement de ce résidu par l'eau et le séchage de la suspension aqueuse ainsi obtenue à poids constant, caractérisé en ce que la concentration de ladite solution aqueuse alcoolique ou cétonique est réalisée en présence de célite.

2. Procédé selon la revendication 1, caractérisisé en ce que le volume de la solution aqueuse obtenue par concentration de la solution aqueuse alcoolique ou cétanique en présence de célite est 1,5 à 1,6 fois supérieur au poids des feuilles traitées.